Europäisches Patentamt

European Patent Office

Office européen des brevets

(19)

(11)  EP 1 447 045 A1

(12)  ## EUROPEAN PATENT APPLICATION

(43) Date of publication:
**18.08.2004 Bulletin 2004/34**

(51) Int Cl.7: **A61B 5/0476**, A61B 5/04

(21) Application number: **03028545.6**

(22) Date of filing: **11.12.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK**<br><br>(30) Priority: **17.02.2003 JP 2003038497**<br>　　　　　　**02.05.2003 JP 2003127206**<br>　　　　　　**13.06.2003 JP 2003170121**<br><br>(71) Applicant: **Brain Functions Laboratory, Inc.**<br>**Kawasaki-shi, Kanagawa 213-0012 (JP)** | (72) Inventors:<br>• **Musha, Toshimitsu,**<br>　**Brain Functions Laboratory Inc.**<br>　**Kawasaki-shi Kanagawa 213-0012 (JP)**<br>• **Take, Noriyuke**<br>　**Kawasaki-shi Kanagawa 213-001 (JP)**<br>• **Mochizuki, Yusuke**<br>　**Machida-shi 194-0004 Tokyo (JP)**<br><br>(74) Representative:<br>**Riederer Freiherr von Paar zu Schönau, Anton**<br>**Boehmert & Boehmert**<br>**Kanzlei Landshut**<br>**Postfach 26 64**<br>**84010 Landshut (DE)** |

(54)  **Method and apparatus for measuring the degree of neuronal impairment in brain cortex**

(57)  In a method and an apparatus for measuring the degree of neuronal impairment in brain cortex, scalp potentials or magnetic fields of a subject are measured by mounting a plurality of sensors (2) on the head of the subject, the measured scalp potentials or magnetic fields are converted into numerical data to obtain a dipolarity at each sampling, mean values of squared errors, within a fixed time interval, between a scalp potential or a magnetic field by an equivalent dipole at a dipolarity peak emergence time and the measured scalp potentials or magnetic fields, or variances of the squared errors from the mean values are obtained for the sensors, and a contour concerning a distribution of the mean values or the variances on a scalp or a brain surface corresponding thereto is mapped as an output. Alternatively, the squares of potentials sensed by the sensors are averaged for several seconds, and variances of these mean values are obtained for numerous reference persons.

FIG.4

EP 1 447 045 A1

**Description**

Background of the Invention

Field of the Invention

The present invention relates to a method and an apparatus for measuring the degree of neuronal impairment in brain cortex, and in particular to a method and an apparatus for measuring or estimating the degree of neuronal impairment in brain cortex such as a senile dementia disorder. The method per se is not a diagnostic method but rather consists of a measuring process.

Description of the Related Art

With respect to senile dementia, it is statistically said that about 30% of nonagenarians are in dementia. This senile dementia is becoming a serious problem for the coming aging society.

Accordingly, such a dementia disorder should be preferably found as early as possible and treated before it results in a serious state. The measurement (estimation) of the dementia disorder has been conventionally performed by various manual methods as follows:

(1) Hasegawa's Dementia rating Scale (HDS)
(2) National mental research dementia screening test
(3) N type mental function examination
(4) Mental Status Questionnaire (MSQ)
(5) Mini-Mental State Examination (MMSE)
(6) Ezawa's "Clinical judgment criteria of senile intelligence";
(7) Functional Assessment Staging Test (FAST);
(8) Clinical Dementia Rating (CDR);
(9) GBS scale;
(10) N mental state scale for old people (NM scale).

Since the prior art dementia measurement methods as mentioned above adopt a test form in which doctors always examine subjects (patients) in interviews, there have been problems as follows:

① Since a questioner exists, the answer greatly depends on the special human relationship between the questioner and the subject, and is not always objectively and accurately obtained, resulting in variation of the judgment result.
② While the subject is repeating the test, he or she may learn the examination contents, so that the objective judgment result can not be obtained.
③ The subject occasionally refuses to answer.
④ A capability of discriminating initial dementia is low.

Also, in the methods utilizing an SPECT (Single Photon Emission Computing Tomography), a PET, and the like, a radioactive material is injected into a vessel to enable a radiation amount radiated in the brain to be measured, whereby a subject gets exposed to radiation and the diagnosis cost becomes very high.

Accordingly, the applicant of the present invention has already proposed a method and an apparatus for measuring (estimating) the degree of neuronal impairment in brain cortex (see e.g. patent document 1) which are inexpensive, non-invasive, highly sensitive, highly reliable, and easy to operate. These method and apparatus will now be described.

When neurons within a brain cortex are in action, an electromotive force is generated, a current flows in a direction perpendicular to the brain cortex surface, and a potential distribution is generated on the scalp. This potential distribution can be approximated by a single current dipole assumed in the brain. A dipole having the minimum mean value of squared errors between a potential generated on a sensor position and measured potentials for all of the sensors is called an equivalent dipole.

The equivalent dipole for a brain wave of a limited bandwidth makes a smooth potential distribution on the scalp woven from potentials sensed by the sensor. A dipolarity indicates a degree of an approximation of an equivalent dipole potential, and minimizes the mean value of the squared errors between two kinds of potentials at the sensor position. Accordingly, the dipolarity serves as an indicator of smoothness of the scalp potential. If the neuronal activity within the cortex is even, the dipolarity is close to 1. If unevenness occurs in the neuronal activity, the dipolarity decreases. The decrease of the dipolarity indicates the decrease of the neuronal activity. Since the dipolarity of the narrow-band brain wave nearly periodically fluctuates temporally, the mean value of the peak values are called a mean dipolarity.

It has been made clear that the mean dipolarity has a threshold value, and that a dementia person can be distinguished from a normal person by the threshold value as a border. Accordingly, the dementia, especially Alzheimer's type dementia has been quantifiable, enabling discrimination between the normal person and the dementia person with a certain accuracy of diagnosis.

The peak value of the dipolarity temporally fluctuates, and its standard deviation increases as a neuronal function is impaired. Such a standard deviation has a threshold value, so that when the standard deviation becomes larger than the threshold value, it is possible to diagnose a person as the Alzheimer's type dementia.

A mean dipolarity $D_\alpha$ concerning an alpha wave decreases as the Alzheimer's type dementia progresses. It has been confirmed by the SPECT that cerebral blood flow from bilateral temporal lobes to a parietal lobe decreases with a positive correlation with the mean dipolarity $D_\alpha$. This is a distinctive tendency for the initial Alzheimer's type dementia.

If the head of a subject is supposed to be spherical, the calculation of the dipolarity becomes very simple. By this method, the calculation becomes very simple, few variations occur in a determination result different from an MMSE method, and the cost is significantly reduced compared with the SPECT method and the like, thereby further enhancing a discrimination sensitivity between a normal person and a demented person.

[Patent Document]

Japanese Patent Application Laid-open No. 2002-248087 (Abstract, Fig. 1)

However, there has been a problem that even though a functional impairment can be discriminated by the mean value of overall brain of the subject, in case of the above-mentioned patent document 1, it is not determined in which portion of the head the functional impairment progresses in the absence of position information of the impairment.

Summary of the Invention

It is accordingly an object of the present invention to provide a method and an apparatus for measuring the degree of neuronal impairment in brain cortex capable of indicating a part of a head in which a functional impairment is in progress.

In order to achieve the above-mentioned object, in a method for measuring the degree of neuronal impairment in brain cortex according to the present invention, scalp potentials or magnetic fields of a subject are measured by mounting a plurality of sensors on the head of the subject; the measured scalp potentials or magnetic fields are converted into numerical data to obtain a dipolarity at each sampling; mean values of squared errors, within a fixed time interval, between a scalp potential or a magnetic field by an equivalent dipole at a dipolarity peak emergence time and the measured scalp potentials or magnetic fields for the sensors are obtained; whereby position information of a brain functional impairment can be obtained. Therefore, by interpolating the above-mentioned squared errors concerning the brain functional impairment at sensor positions, a contour (contour line) concerning a distribution of the squared errors on a scalp or a brain surface corresponding thereto is mapped as an output. The evaluation can be carried out by data processing, and carrying out the method can be the job of a technician.

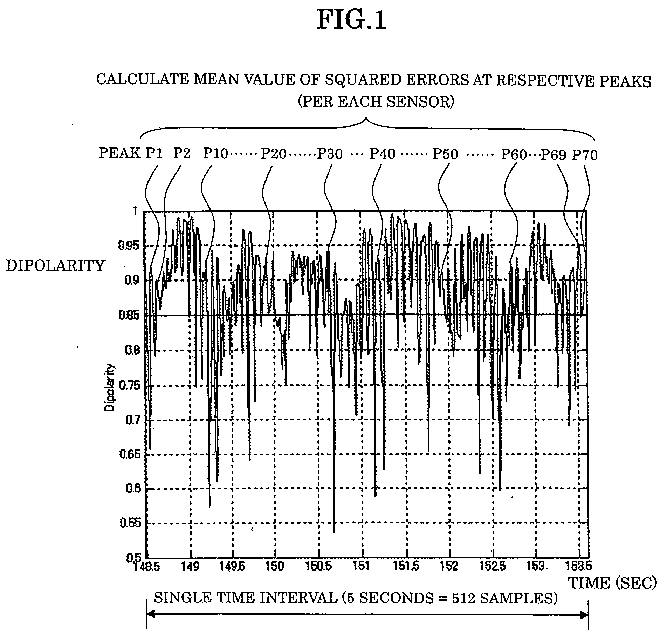

Namely, in the same way as the above patent document 1, a dipolarity as shown in Fig. 1 is obtained from scalp potentials measured by a sensor mounted on the head of a subject. The dipolarity in this case can be obtained for overall sampling times (512 samples in the example of Fig. 1).

However, in the present invention, the thus obtained dipolarity data themselves are not used, instead dipolarities at peak emergence times (P1, P2, ... P70) are obtained. When the dipolarity at each sampling is obtained, squared errors between a scalp potential by an equivalent dipole and the actually measured scalp potentials are already obtained, so that the squared error at each peak emergence point is taken out and a mean value within a fixed time interval is obtained.

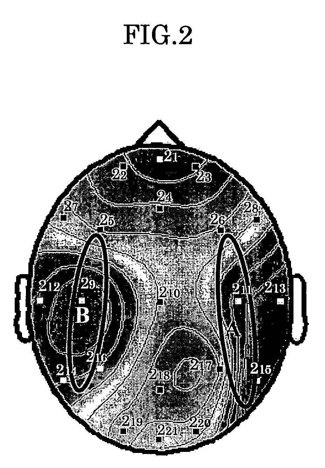

If such a mean value of the squared errors within a designated time interval is obtained for each sensor, the mean value of the squared errors in each sensor positioned within a plane of a head is obtained, so that as shown in Fig. 2 a contour concerning a distribution of the mean values on a scalp or a brain surface corresponding thereto is mapped as an output.

Thus, a spatial aspect of a subject's brain activity in a fixed time interval can be recognized.

Namely, since a current distribution on a brain cortex is uneven in a portion where a neuronal function of the brain cortex is impaired, a deviation of a scalp potential distribution from potential distribution of the equivalent dipole locally becomes larger at that portion. This is indicated by a dark color portion of the contour map obtained as mentioned above.

In this contour map, as the color becomes dark, the value becomes lower. The areas indicated by thick lines A and B (Fig. 2) are portions in which a regional cerebral blood flow obtained by an SPECT is decreased and they coincide with positions with a functionally impaired brain activity

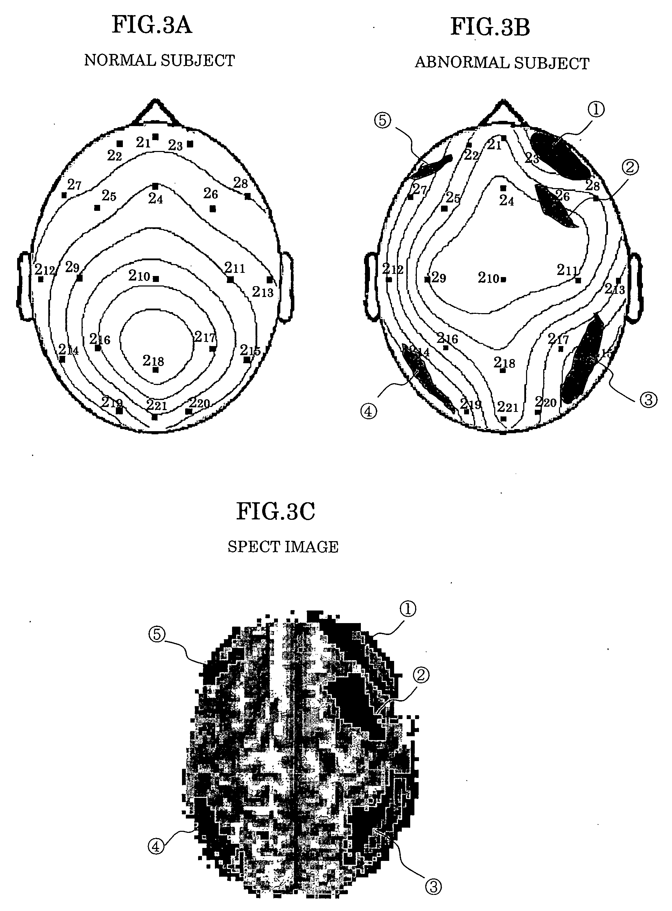

Namely, as shown in Fig. 3A, a contour map obtained from a definitely normal subject mainly assumes an outward smooth convex state. However, if dementia begins, a cortex becomes uneven by a tessellar impairment of cerebral cortex nerve cells as shown in Fig. 3B. As a result, equipotential lines of the scalp potentials assume distorted equipotential line portions ①-⑤ which partially assume the inward convex states.

Fig. 3C shows a SPECT image in which a decreased amount of cerebral blood flow measured by a SPECT for the subject of Fig. 3B is mapped on a reference brain, which corresponds to the portions ①-⑤ assuming the inward convex states shown in Fig. 3B.

Thus, since the dipolarity for the measured potential at each sampling time fluctuates, the peak dipolarity values are captured so that the squared error between the measured value at each sensor position at the peak emergence time and the dipole potential is computed to map a contour. This contour map indicates a local inactivation of the neuronal activity. Since this contour map temporally fluctuates, a temporal mean value of the squared errors within a given fixed time interval is obtained to map a contour concerning a distribution on a scalp or a brain surface corresponding thereto in the above-mentioned invention.

On the other hand, if a local impairment of a brain function due to e.g. brain impairment occurs, the temporal fluctuation of the contour map is small since the impaired position is fixed. Accordingly, an impairment due to Alzheimer's disease can not be distinguished from that due to blood vessel dementia by the brain impairment only by the mean value. However, they can be distinguished by observing the move of the contour map. In order to observe an existence of the movement, instead of the mean value of the squared errors at the sensor position, a variance (distribution) indicating a variation of the squared errors around the mean value may be used.

While the local functional impairment of neurons is detected from the contour map of the mean values, when the variance value is small, it can be determined that there is a high possibility of the local brain functional impairment due to the brain impairment.

It is to be noted that as for a method for mapping a potential distribution on a scalp to a brain cortex, a known method may be used.

Besides the above-mentioned method for measuring the degree of neuronal impairment in brain cortex by using the dipolarity or the equivalent dipole, it is possible in the present invention to map a contour by using a concept of a standard deviation of the mean squared potential measured at each sensor to measure the degree of neuronal impairment in brain cortex. Impaired neuronal activity becomes either more or less unstable than that of a normal neuron, and the degree of instability of scalp potential can be an indicator of cortical neuronal activity.

For this reason, the present invention provides a method for measuring the degree of neuronal impairment in brain cortex comprising the steps of: measuring scalp potentials or magnetic fields by a plurality of sensors mounted on the heads of reference persons, to be converted into numerical data; obtaining squared mean values from the numerical data at the sensors for every short time interval within a designated measurement time; obtaining a normalized standard deviation of the squared mean values within the designated measurement time; obtaining the normalized standard deviations for a plurality of reference persons; obtaining a mean value of the normalized standard deviations and an inter-reference person standard deviation for the mean value to be stored; and determining a distance (gap) level of a normalized standard deviation similarly obtained for a subject from the normalized standard deviation mean value of the reference persons based on the inter-reference person standard deviation, made different between a positive side and a negative side of the normalized standard deviation mean value of the reference persons.

Namely, in the present invention, brain wave data of reference persons (e.g. persons whose brain function is recognized to be normal) are preliminarily obtained, and by comparing the data with the brain data obtained for the subject in connection with the standard deviation, the degree of neuronal impairment in brain cortex is determined.

For this determination, a plurality of sensors are firstly mounted on the heads of the reference persons as mentioned above to obtain numerical data from the scalp potentials measured by the sensors.

Based on the numerical data, data described below are obtained for each sensor to be stored.

Namely, from the numerical data, squared mean values are obtained for a short time interval within a fixed measurement time (designated interval), and a normalized (specified) standard deviation of the squared mean values within the above-mentioned designated time is obtained. The normalized standard deviation is obtained, such that the squared mean values for the above-mentioned short time interval are averaged within the designated time, and the standard deviation around the mean value is obtained, and the standard deviation is divided by the above-mentioned mean value. It is to be noted that not the standard deviation itself but the normalized standard deviation is obtained for eliminating an influence of amplitudes of brain waves.

Since the normalized standard deviation thus obtained is for a single reference person, the normalized standard deviations are obtained for numerous reference persons, and the mean value for the normalized standard deviations and a standard deviation for the mean value (inter-reference person standard deviation) are obtained and stored.

After the reference data of the reference persons are thus obtained, comparison data for the subject are obtained.

Namely, squared mean values are similarly obtained for the subject for a short time interval within a designated measurement time, and the normalized standard deviation of the squared mean values within the designated measurement time is obtained.

A distance level of a normalized standard deviation thus obtained for a subject from the mean value of the normalized standard deviations of the reference persons obtained as mentioned above is determined based on the above-mentioned inter-reference person standard deviation.

In this case, the distance levels of the normalized standard deviation for the subject from the normalized standard deviation mean value of the reference persons are made different between the positive side and the negative side of the mean value.

Thus, as the normalized standard deviation for the subject becomes farther from the normalized standard deviation mean value of the reference persons, it can be identified that it is a portion where a neuronal activity is abnormally unstable (in case of positive side of the mean value) than the reference persons, or that it is an abnormally stable (less active) portion (in case of negative side of the mean value).

It is to be noted that the levels may be projected to positions on the brain surface preliminarily obtained corresponding to sensor positions on the scalp, and by interpolating the levels, a contour concerning a distribution on the brain surface can be mapped and colored.

Thus, the squares of potentials sensed by the sensors are averaged for several seconds, and variances of these mean values are obtained for numerous reference persons. The mean value and the standard deviation of the variances for a group of the reference persons are obtained, so that the variance of an individual subject is ranked depending on which area of the standard deviation of the group of the reference persons the variance is located. By allocating the values to a brain surface corresponding to the sensors, maps are prepared. By these maps, a local impairment degree of a neuronal function is indicated.

An apparatus for realizing the above-mentioned method for measuring the degree of neuronal impairment in brain cortex according to the present invention may comprise: a plurality of sensors to be mounted on the head of a subject for measuring scalp potentials or magnetic fields of the subject; a computing unit for converting output signals of the sensors into numerical data to obtain a dipolarity at each sampling, for obtaining mean values of squared errors, within a fixed time interval, between a scalp potential or a magnetic field by an equivalent dipole at a dipolarity peak emergence time and the measured scalp potentials or magnetic fields or variances of the squared errors from the mean values from the sensors, and for mapping a contour concerning a distribution of the mean values or the variances on a scalp or a brain surface corresponding thereto; and an output unit for outputting a contour map.

Also, another apparatus for measuring the degree of neuronal impairment in brain cortex according to the present invention comprises: a plurality of sensors to be mounted on the heads of reference persons or a subject for measuring scalp potentials or magnetic fields; and a computing unit for converting the scalp potentials or magnetic fields of the reference persons into numerical data, for obtaining squared mean values from the numerical data at the sensors for every short time interval within a designated measurement time, for obtaining a normalized standard deviation of the squared mean values within the designated measurement time, for obtaining the normalized standard deviation for a plurality of reference persons, for obtaining a mean value of the normalized standard deviation and an inter-reference person standard deviation for the mean value to be stored, and for determining a distance level of a normalized standard deviation similarly obtained for a subject from the normalized standard deviation mean value of the reference persons based on the inter-reference person standard deviation, made different between a positive side and a negative side of the normalized standard deviation mean value of the reference persons.

It is to be noted that the above-mentioned computing unit may project levels to positions, on a brain surface, preliminarily obtained corresponding to sensor positions on a scalp, and may map and color a contour concerning a distribution on the brain surface by interpolating the levels.

Also, in the present invention, a program for making a computer execute may be provided. This program for measuring the degree of neuronal impairment in brain cortex comprises procedures of obtaining a dipolarity at each sampling based on numerical data of scalp potentials of a subject measured by mounting a plurality of sensors on the head of the subject; obtaining mean values of squared errors, within a fixed time interval, between a scalp potential by an equivalent dipole at a dipolarity peak emergence time and the measured scalp potentials, or variances of the squared errors from the mean values for the sensors; and mapping a contour concerning a distribution of the mean values or the variances on a scalp or a brain surface corresponding thereto, as an output.

Furthermore, the present invention provides a computer program for measuring the degree of neuronal impairment in brain cortex, and making a computer execute the steps of: measuring scalp potentials by a plurality of sensors mounted on the heads of reference persons, to be converted into numerical data; obtaining squared mean values from the numerical data at the sensors for every short time interval within a designated measurement time;

obtaining a normalized standard deviation of the squared mean values within the designated measurement time; obtaining the normalized standard deviation for a plurality of reference persons; obtaining a mean value of the normalized standard deviations and an inter-reference person standard deviation for the mean value to be stored; and determining a distance level between a normalized standard deviation similarly obtained for a subject from the normalized standard deviation mean value of the reference persons based on the inter-reference person standard deviation, made different between a positive side and a negative side of the normalized standard deviation mean value of the reference persons.

In this program, levels may be projected to positions, on a brain surface, preliminarily obtained corresponding to sensor positions on a scalp, and by interpolating the levels, a contour concerning a distribution on the brain surface may be mapped and colored.

Furthermore, the present invention provides a computer readable recording medium recorded with the above-mentioned program.

It is to be noted that the mean values or the variances within the fixed time interval may be obtained for an overall time interval, and a contour for the mean values or the variances may be mapped as an output.

Furthermore, an approximate degree, after a predetermined frequency component within the data is extracted, and based on the predetermined frequency component, at a time when one or more equivalent dipoles are determined in which a mean value of a squared error between a potential distribution which one or more current dipoles, supposed in the head, form at positions of the sensors and a measured potential of the sensors indicated by the data becomes least, may be used as the dipolarity. The head in this case may adopt a spherical model.

Also, as the above-mentioned sensors, EEG sensors or MEG sensors may be used.

The scalp potentials may be detected by a terminal equipment, the data may be transmitted to an operation center through a communication line, and the operation center may prepare the map from the data to be returned to the terminal equipment through the communication line.

Brief Description of the Drawings

The above and other objects and advantages of the invention will be apparent upon consideration of the following detailed description, taken in conjunction with the accompanying drawings, in which the reference numerals refer to like parts throughout and in which:

Fig. 1 is a graph indicating a dipolarity when a head is assumed to be spherical and a single equivalent dipole is used in order to realize a method and an apparatus for measuring the degree of neuronal impairment in the brain cortex, as well as a program and a recording medium thereof according to the present invention;

Fig. 2 is a contour map of a scalp potential obtained by the present invention;

Figs. 3A-3C are diagrams contrasting contour maps of scalp potentials obtained by the present invention and an SPECT image;

Fig. 4 is a block diagram showing an embodiment of the present invention;

Fig. 5 is a block diagram showing a modification of the present invention;

Fig. 6 is a flow chart showing a processing procedure example (1) of a computing unit used for the present invention;

Fig. 7 is a flow chart showing a processing procedure example (2) of a computing unit used for the present invention;

Fig. 8 is a sectional side view of a head showing sensor projection points for a distribution on a brain surface by the above processing procedure (2); and

Figs. 9A-9C are diagrams showing a distribution on a brain surface at each level by the above processing procedure (2).

Description of the Embodiments

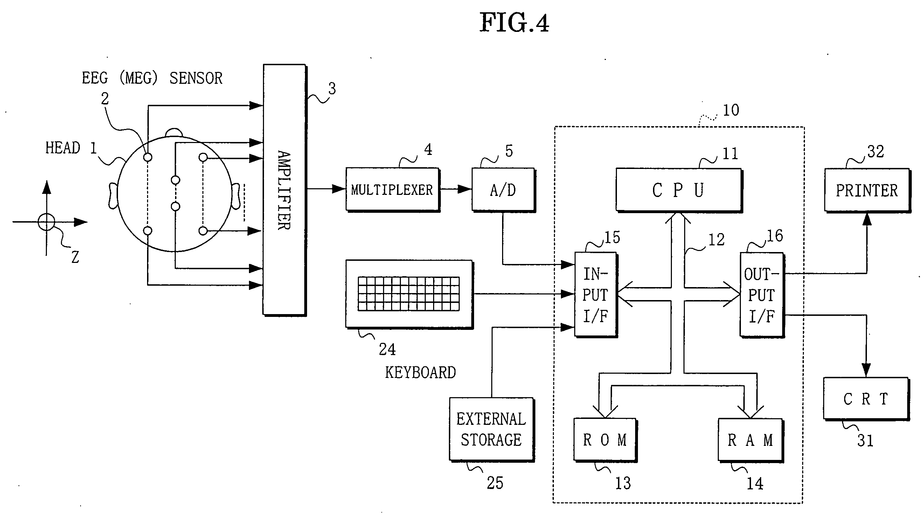

Fig. 4 shows one embodiment of a method and an apparatus for measuring the degree of neuronal impairment in the brain cortex according to the present invention.

In this embodiment, a group of EEG sensors or MEG sensors 2, in the example $2_1$-$2_{21}$ comprising e.g. twenty-one sensors, is firstly mounted on a head 1 to measure scalp potentials, or a subject puts on a cap where the sensors are properly arranged. It is to be noted that the sensors 2 in this case may be arranged according to the international 10-20 standard.

The measured potential from the sensors 2 is supplied, through an amplifier 3 and a multiplexer 4, to an analog/digital (A/D) converter 5, so that the digitized measured potential (EEG) data are supplied to a computer 10, in which are connected through a bus 12 a CPU 11, an ROM 13, an RAM 14, an input interface 15, and an output interface 16, through the input interface (I/F) 15. It is to be noted that after the input interface 15 has taken out only the component having a frequency band (e.g. alpha wave) preliminarily designated, by performing a digital filtering process, the following processing can be performed to the data.

The above-mentioned ROM 13 is a medium storing the above-mentioned program and the like for determining the equivalent dipole, and the RAM 14 is a memory for storing EEG data from a digitizer 23, a keyboard 24, and the A/D converter 5. An external storage 25 stores characteristic data of the graphs shown in Figs. 1 and 2. The output interface 16 is connected to a CTR 31 and a printer 32.

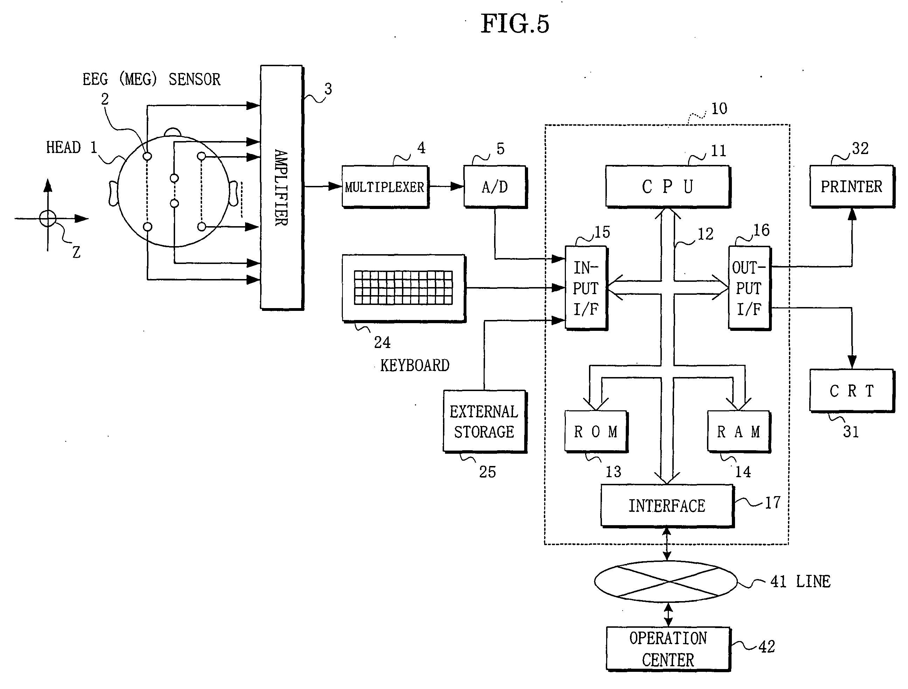

It is to be noted that also such an arrangement as shown in Fig. 5 and described in the following may be employed: The brain wave data are sent from an interface 17 of the computer 10 serving as a data transfer terminal equipment only in this case through a communication line 41 of the Internet or the like to an operation center 42, as a computing (arithmetic) unit, where the result analyzed at the operation center 42 is again sent back to the computer 10 in the clinical spot through the communication line 14, and the result is outputted to an output unit such as the CRT 31 and the printer 32, so that the result can be used as the materials for evaluation. In this case, the program and the recording medium are provided in the operation center.

The external storage 25 is connected to the input interface 15. The display of the CRT 31 or the like which displays the operation result (MMSE value as the dementia degree) of the computer 10 and the printer 32 printing the data and the waveform displayed at the display 31 are connected to the output interface 16 as output units. It is to be noted that all of the programs and the like may be stored only in the ROM 13 without using the external storage 25.

Processing example (1)

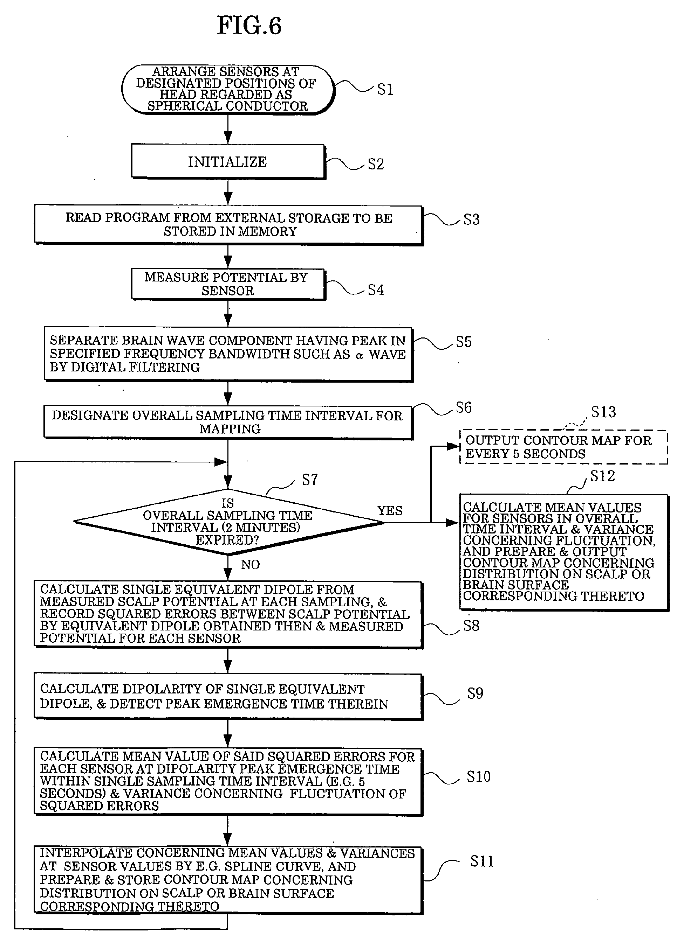

The operation of the embodiments in the above-mentioned arrangement will now be described referring to the flow chart shown in Fig. 6.

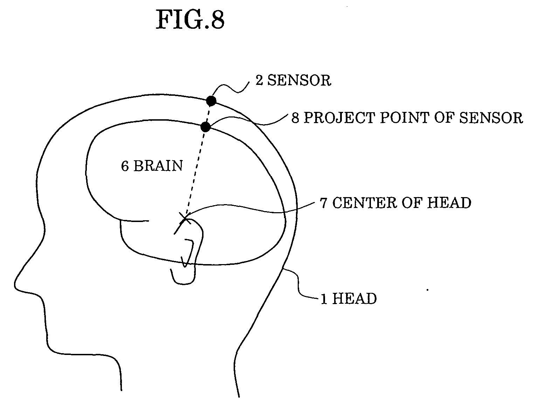

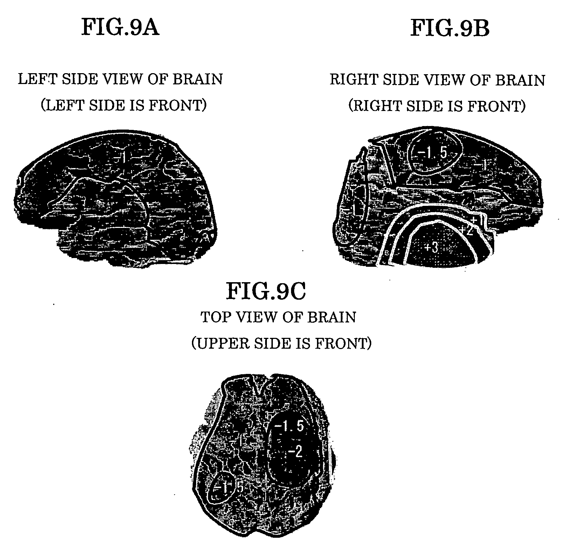

After the sensors 2 are firstly arranged (at step S1) on the head 1 as shown in Figs. 8 and 9, the computer 10 is initialized with a power source (not shown) being made "on" (at step S2).

Then, the programs for various operations, those for signal processing and the like, are read out of the external storage 25 for being stored in the RAM 14 of the computer 10 (at step S3). Such programs may be preliminarily stored in the ROM 13 that is a nonvolatile memory in the computer 10.

Then, the potential measurement based on the neuronal activation in the brain is performed at a fixed sampling interval by the twenty-one sensors $2_1$-$2_{21}$ mounted on the head 1 (at step S4). In the example of Fig. 1, 512 samples are obtained at intervals of approximately 10 ms for a fixed time interval of 5 seconds, which is repeated for 2 minutes.

Hereafter, the EEG component having the peak in the specific frequency bandwidth such as the alpha band is separated by the digital filtering process (at step S5).

Then, an overall sampling time interval is designated for mapping (at step S6). This means as mentioned above that a single sampling time interval (5 seconds) shown in Fig. 1 is designated to be repeated for 2 minutes.

Hereafter, whether or not the overall sampling time interval (2 minutes) has expired is determined (at step S7), and while it has not expired, the following steps S8-S11 are repeatedly executed.

Firstly, at step S8, the equivalent dipoles for all of the sampled measured potentials on the scalp are calculated.

Namely, at step S8, as mentioned above, the CPU 11 of the computer 10 calculates the potential ($V_c$) at the electrode positions on the scalp generated by e.g. a single current dipole in case the current dipole is supposed to be placed at a predetermined position at the head, the mean value of the squared error with the potential ($V_m$) measured at step S4 is obtained, the position and the moment of the current dipole which make the mean value of the squared error are obtained, and such processes are repeated until the squared error converges to be equal to or less than a reference value. When the squared error converges to become equal to or less than the reference value, the current dipole at the position is made an equivalent dipole to store the position in the RAM 14.

Squared errors obtained by the above-mentioned computation process are also stored in the RAM 14. This is performed for each of the sensors $2_1$-$2_{21}$.

Hereafter, the dipolarity is calculated (at step S9). Namely, a dipolarity "d" (not yet the mean value at this point) indicating to what extent the equivalent dipole approximates to the measured potential is calculated as shown in the following equations (1) and (2).

$$d = \sqrt{1 - \frac{\sum_{i=1}^{M}(V_{mi} - V_{ci})^2}{\sum_{i=1}^{M} V_{mi}^2}} \qquad \text{...Eq.(1)}$$

$$d = \sqrt{1 - \frac{\left\{\sum_{i=1}^{M}(V_{mi}-V_{ma})(V_{ci}-V_{ca})\right\}^2}{\sum_{i=1}^{M}(V_{mi}-V_{ma})^2 \sum_{i=1}^{M}(V_{ci}-V_{ca})^2}} \qquad \text{...Eq.(2)}$$

It is to be noted that in the above-mentioned equations (1) and (2), M indicates the number of the sensor 2, and $V_{ma}$ and $V_{ca}$ respectively indicate the mean values of the measured values and calculated values. As for a normal subject, the dipolarity "d"=1.

Thus, peak values of the dipolarity values "d" obtained for each of the measured potentials on the scalp sampled per 10 ms are detected (at step S8). This can be detected by pre-storing peaks P1-P70 having emerged within a single sampling time interval of 5 seconds, as shown in Fig. 1, in the RAM 14 after 5 seconds have expired.

The above-mentioned squared errors at peak emergence times are read from the RAM 14 to obtain the mean value of the sum for each sensor within a single sampling time interval (5 seconds) (at step S10).

Namely, by using the squared errors, obtained in the calculating process of the equivalent dipole, between the measured potentials by the twenty-one sensors $2_1$-$2_{21}$, and the scalp potential by the equivalent dipole, the mean value for each sensor within a single time interval is calculated. Namely, by referring to the example of Fig. 1, the mean value of the squared errors respectively obtained for the peaks P1-P70 is calculated for each sensor.

It is to be noted that the variance of the squared errors from the mean value within the fixed time interval (5 seconds) may be substituted for the mean value, as mentioned above. The same applies to the followings.

Thus, the mean values (variances) of the squared errors between the scalp potentials measured by the twenty-one sensors $2_1$-$2_{21}$ and the scalp potentials by the equivalent dipole are obtained. Therefore, these mean values (variances) between sensors are interpolated by e.g. a spline curve or a bilinear curve to prepare a contour map (at step S11). The lines existing between the sensors in Fig. 2 indicate contours, which are stored (at step S11).

Thus, when the procedure returns to step S7 after the contour map for the single time interval is obtained, unless the overall time interval has expired, the steps S8-S11 are repeated. When the overall time interval has expired, the contour map as shown in Fig. 2 is also prepared, as an output, for the mean value (variance) for each sensor within the overall time interval (at step S12).

It is to be noted that since the contour map is already prepared for every 5 seconds before the overall time interval expires, this can be outputted in a different way from step S12, as shown by step S13 of a dotted line.

Namely, a defect portion of a neuronal function stays in a specific part of a brain, but temporally fluctuates. The information concerning brain disease is included in the manner of the fluctuation. Therefore, the movement of an equipotential map can be displayed in an electronic chart as animation.

It is to be noted that while twenty-one sensors (electrodes) are used in the above-mentioned embodiment, the accuracy of localization can be improved if the number of electrodes is increased. Also, it is possible to project the scalp potential distribution on the brain cortex and to make a similar diagram on the scalp. In this case, an inverse matrix of a transfer matrix which converts the potential distribution on e.g. the brain cortex into the potential of the electrode position designated on the scalp may be used.

Also, the above-mentioned computation can be performed by offline processing by saving all data on files.

Processing example (2)

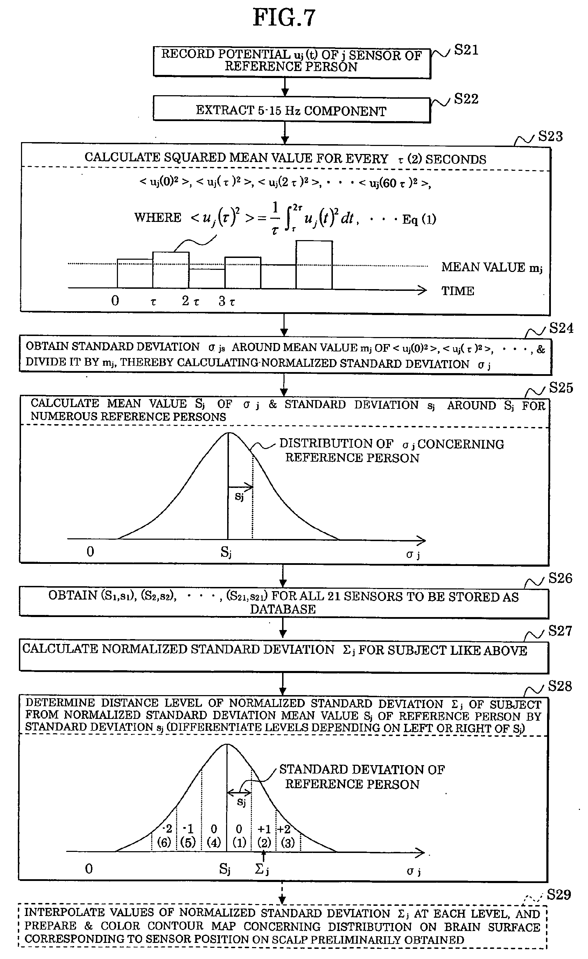

Hereafter, an embodiment for preparing a contour map without using the dipolarity or the equivalent dipole as mentioned above will be described by referring to a flow chart shown in Fig. 7.

Firstly, in the same way as step S4 shown in Fig. 6, potentials are measured by the sensors (electrodes) 2. Although there are e.g. twenty-one sensors in this case, the potentials are independently measured at each sensor in the flow chart of Fig. 7.

Accordingly, taking the "j" sensor among the twenty-one sensors as an example, a scalp potential $u_j(t)$ of a reference (normal) person at the "j" sensor is measured and recorded (at step S21).

Hereafter, in the same way as step S5 of Fig. 6, the frequency components of e.g. 5 to 15Hz are extracted by the digital filter (at step S22).

The numerical data thus obtained are firstly sectioned every $\tau$ seconds (e.g. 2 seconds) within a designated time (e.g. 2 minutes) to calculate the squared mean values (at step S23).

The squared mean values $<u_j(0)^2>$, $<u_j(\tau)^2>$, $<u_j(2\tau)^2>$, ... $<u_j(60\tau)^2>$ in this case are calculated, as shown in Fig. 7, based on the equation (1) at step S23 in Fig. 7, for e.g. 400 samples extracted for every 2 seconds.

Then, a mean value $m_j$ of the squared mean values $<u_j(0)^2>$, $<u_j(\tau)^2>$, $<u_j(2\tau)^2>$, ... $<u_j(60\tau)^2>$ is obtained, a standard deviation $\sigma_{js}$ around the mean value $m_j$ is obtained, and a normalized standard deviation $\sigma_j$ is calculated by dividing the standard deviation $\sigma_{js}$ by the above mean value $m_j$ (at step S24).

It is to be noted that the reason why the obtained standard deviation $\sigma_{js}$ is divided by the mean value $m_j$ in this way is to exclude influences of brain wave amplitudes, and to normalize respective brain data.

The normalized standard deviation $\sigma_j$ thus obtained is brain data for only a single reference person. Therefore, in order to improve the accuracy of data, the normalized standard deviations $\sigma_j$ for numerous reference persons are obtained, and a mean value $S_j$ of the standard deviations $\sigma_j$, and a standard deviation $S_j$ (inter-reference person standard deviation) around the mean value $S_j$ are calculated (at step S25).

Thus, as shown at step S25 of Fig. 7, it becomes possible to indicate the mean value $S_j$ and the standard deviation $S_j$ in the distribution of the normalized standard deviation $\sigma_j$ concerning the reference persons.

Since the data obtained so far are those for a single "j" sensor, combinations $(S_1, s_1)$, $(S_2, s_2)$, ... $(S_{21}, s_{21})$ of the mean value $S_j$ and the standard deviation $S_j$ for all of the sensors (e.g. 21 sensors as mentioned above) are obtained and stored as a basic database (at step S26). However, as mentioned above, processing is performed per each sensor in this invention.

Hereafter, the procedure proceeds to a data estimation of a subject's brain wave.

Namely, a normalized standard deviation $\Sigma_j$ for the subject is also calculated through the above mentioned steps S21-S24 (at step S27).

Then, to what extent the thus obtained normalized standard deviation $\Sigma_j$ of the subject is distant from the mean value $S_j$ of the normalized standard deviation of normal persons obtained at the above-mentioned step S25 or the distance of the former from the latter, and whether the normalized standard deviation $\Sigma_j$ is on the positive side or the negative side of the mean value $S_j$ are determined based on the standard deviation $S_j$ (at step S28).

Namely, as illustrated at step S28 in Fig. 7, levels (1) to (6) indicating a distance level of the normalized standard deviation $\Sigma_j$ of the subject from the normalized standard deviation $S_j$ of the reference persons, which is centered, can be determined per standard deviation $S_j$.

In case of the example shown at step S28, the section (1) of $S_j < \Sigma_j < S_j + s_j$ is made a level 0 at which abnormality is not specifically recognized. As $\Sigma_j$ moves away from $S_j$, the estimations assume as follows:

$S_j + s_j \leq \Sigma_j < S_j + 2s_j$ ... level + 1 (section (2): section shown by an arrow)

$S_j + 2s_j \leq \Sigma_j < S_j + 3s_j$ ... level +2 (section (3))

.....

Accordingly, as the level on the positive side increases, $\Sigma_j$ moves away from the mean value $S_j$ of the reference persons. Therefore, a person in such a case can be determined to have a larger instability of the brain wave than the instability of the reference persons, and to be in an abnormal state.

On the other hand, as for the sections (4), (5), (6) ... on the negative side of the mean value $S_j$, the section (4) is supposed to be level 0 on which no abnormality is found like the section (1).

As $\Sigma_j$ moves away from $S_j$, the estimations are as follows:

$S_j - \sigma \geq \Sigma_j > S_j - 2\sigma$ ... level -1 (section (5))

$S_j - 2\sigma \geq \Sigma_j > S_j - 3\sigma$ ... level -2 (section (6))

.....

In this case, as the distance level on the negative side increases, the instability of the brain wave becomes less than that of the reference persons. However, since $\Sigma_j$ moves away from the mean value $S_j$ of the reference persons, this can be also recognized as an abnormal state.

Thus, the level of the brain activity can be estimated. This can be generalized as follows:

The sensor satisfying the relationship of $S_j + as_j < \Sigma_j < S_j + (a+1)s_j$ is assigned a level "a" on the positive side, for a=0, 1, 2, 3, .... Also, the sensor corresponding to $S_j - bs_j < \Sigma_j < S_j - (b+1)s_j$ can be assigned a level "b" on the negative side.

It is also possible that values of the normalized standard deviation $\Sigma_j$ are interpolated on respective levels, and a contour map concerning a distribution on the brain surface is colored, in the same way as the above-mentioned embodiment (at step S29). For this reason, it is possible to project a sensor position on the scalp on a surface of a standard brain. For example, as shown in Fig. 8, the point where the line between the center (position between temples) of a head 1 and a scalp electrode 2 intersects a surface of a brain 6 may be supposed to be a projection value of the electrode 2.

Namely, the values of the normalized standard deviation $\Sigma_j$ of the sensor to which the positive side level "a" is allocated are interpolated to prepare a map colored e.g. red. Also, the values of the normalized standard deviation $\Sigma_j$ of the sensor to which the negative side level "b" is allocated are interpolated to prepare a map colored e.g. blue. By darkening the colors according to the values of "a" and "b", it becomes possible to project the sensor position on the scalp, as shown in Figs. 9A-9C, on a brain surface.

Thus, the "red" area as defined above is an area (area away from $S_j$ to the positive side) where the instability

EP 1 447 045 A1

of the neuronal activity is large, the "blue" area as defined above is an area (area away from $S_j$ to the negative side) where the neuronal activity is more stable than the normal person so that the information unobtainable by the SPECT can be easily obtained.

For example, as for an anti-dementia medicine, the difference of effects between the red area and the blue area may clearly appear in some cases. This is expected to come into effect in future dementia therapy by a drug therapy and rehabilitation.

Thus, the neuronal activity is supported by a synapse activity, so that there is a possibility of separating the function of the synapse itself and the function of a neuronal cyton. It becomes possible to obtain new information about a brain activity which has not been observed invasively and directly.

As described above, a method and an apparatus for measuring the degree of neuronal impairment in brain cortex, as well as a program and a recording medium therefor according to the present invention are arranged so that: scalp potentials or magnetic fields of a subject are measured by mounting a plurality of sensors on the head of the subject, the measured scalp potentials or magnetic fields are converted into numerical data to obtain a dipolarity at each sampling; mean values of squared errors, within a fixed time interval, between a scalp potential or a magnetic field by an equivalent dipole at a dipolarity peak emergence time and the measured scalp potentials or magnetic fields or variances of the squared errors from the mean values are obtained for the sensors; and a contour concerning a distribution of the mean values or the variances on a scalp or a brain surface corresponding thereto is mapped as an output. Therefore, two-dimensional information of a head can be obtained, thereby enabling a position of a functional impairment to be specified.

Accordingly, an effect at each portion of a brain can be estimated in a short time, and temporal fluctuant information is also obtained. Therefore, the present invention can be adopted to determine disorder whose symptom temporally changes like "partial-senile". Accordingly, there is a possibility for distinguishing a neuronal functional defect from a synapse functional defect.

Furthermore, a contour concerning a distribution on a scalp or a brain surface corresponding thereto is mapped by using the variances from the mean values of the squared errors between a scalp potential or a magnetic field by an equivalent dipole at a dipolarity peak emergence time and the measured scalp potentials or magnetic fields within a fixed time interval, whereby an impairment due to Alzheimer's disease can not be distinguished from that due to blood vessel dementia by the brain impairment.

Furthermore, from numerical data of numerous reference persons, a normalized standard deviation mean value of the reference persons and an inter-reference person standard deviation for the mean value are obtained, and a distance level of a normalized standard deviation for a subject based on the numerical data from the normalized standard deviation mean value of the reference persons is determined based on the inter-reference person standard deviation, made different between a positive side and a negative side of the normalized standard deviation mean value of the reference persons, thereby enabling an unstable area of a neuronal activity or an area where a neuronal activity is more stable than a normal person but an abnormality is recognized to be distinguished.

**Claims**

1. A method for measuring the degree of neuronal impairment in brain cortex, comprising the steps of:

measuring scalp potentials or magnetic fields of a subject (1) by mounting a plurality of sensors (2) on the head of the subject;
converting the measured scalp potentials or magnetic fields into numerical data to obtain a dipolarity at each sampling;
obtaining mean values of squared errors, within a fixed time interval, between a scalp potential or a magnetic field by an equivalent dipole at a dipolarity peak emergence time and the measured scalp potentials or magnetic fields, or variances of the squared errors from the mean values for the sensors; and
mapping a contour concerning a distribution of the mean values or the variances on a scalp or a brain surface corresponding thereto, as an output.

2. The method for measuring the degree of neuronal impairment in brain cortex as claimed in claim 1, wherein the mean values or the variances within the fixed time interval are obtained for an overall time interval, and a contour for the mean values or the variances is mapped as an output.

3. The method for measuring the degree of neuronal impairment in brain cortex as claimed in claim 1 or 2, wherein the head comprises a spherical model.

**4.** A method for measuring the degree of neuronal impairment in brain cortex, comprising the steps of:

measuring scalp potentials or magnetic fields by a plurality of sensors mounted on the heads of reference persons, to be converted into numerical data;

obtaining squared mean values from the numerical data at the sensors for every short time interval within a designated measurement time;

obtaining a normalized standard deviation of the squared mean values within the designated measurement time;

obtaining the normalized standard deviations for a plurality of reference persons;

obtaining a mean value of the normalized standard deviations and an inter-reference person standard deviation for the mean value to be stored; and

determining a distance level of a normalized standard deviation similarly obtained for a subject from the normalized standard deviation mean value of the reference persons based on the inter-reference person standard deviation, made different between a positive side and a negative side of the normalized standard deviation mean value of the reference persons.

**5.** The method for measuring the degree of neuronal impairment in brain cortex as claimed in claim 4, wherein levels are projected to positions, on a brain surface, preliminarily obtained corresponding to sensor positions on a scalp, and by interpolating the levels, a contour concerning a distribution on the brain surface is mapped and colored.

**6.** The method for measuring the degree of neuronal impairment in brain cortex as claimed in any of claims 1 to 5, wherein the sensors (2) comprise EEG sensors or MEG sensors.

**7.** The method for measuring the degree of neuronal impairment in brain cortex as claimed in any one of claims 1 to 6, wherein the scalp potentials or the magnetic fields are detected by a terminal equipment (10), the data are transmitted to an operation center (42) through a communication line (41), and the operation center prepares the map from the data to be returned to the terminal equipment through the communication line.

**8.** An apparatus for measuring the degree of neuronal impairment in brain cortex, comprising:

a plurality of sensors (2) to be mounted on the head (1) of a subject for measuring scalp potentials or magnetic fields of the subject;

a computing unit (10) adapted for converting output signals of the sensors into numerical data to obtain a dipolarity at each sampling, for obtaining mean values of squared errors, within a fixed time interval, between a scalp potential or a magnetic field by an equivalent dipole at a dipolarity peak emergence time and the measured scalp potentials or magnetic fields or variances of the squared errors from the mean values from the sensors, and for mapping a contour concerning a distribution of the mean values or the variances on a scalp or a brain surface corresponding thereto; and

an output unit (31, 32) for outputting a contour map.

**9.** The apparatus for measuring the degree of neuronal impairment in brain cortex as claimed in claim 8, wherein the computing unit (10) obtains the mean values or the variances within the fixed time interval for an overall time interval, and maps a contour for the mean values or the variances.

**10.** The apparatus for measuring the degree of neuronal impairment in brain cortex as claimed in claim 8 or 9, wherein the head (1) comprises a spherical model.

**11.** An apparatus for measuring the degree of neuronal impairment in brain cortex, preferably according to claim 8 or 9, comprising:

a plurality of sensors (2) to be mounted on the heads (1) of reference persons or a subject for measuring scalp potentials or magnetic fields; and

a computing unit (10) adapted for converting the scalp potentials or magnetic fields of the reference persons into numerical data, for obtaining squared mean values from the numerical data at the sensors for every short time interval within a designated measurement time, for obtaining a normalized standard deviation of the squared mean values within the designated measurement time, for obtaining the normalized standard deviation for a plurality of reference persons, for obtaining a mean value of the normalized standard deviation and an inter-reference person standard deviation for the mean value to be stored, and for determining a distance level of a normalized standard deviation similarly obtained for a subject from the normalized standard deviation mean value of the reference persons based on the inter-reference person standard deviation, made different between a positive side and a negative side of the normalized standard deviation mean value of the reference persons.

**12.** The apparatus for measuring the degree of neuronal impairment in brain cortex as claimed in any of claims 8 to 11, wherein the computing unit projects levels to positions, on a brain surface, preliminarily obtained corresponding to sensor positions on a scalp, and maps and colors a contour concerning a distribution on the brain surface by interpolating the levels.

**13.** The apparatus for measuring the degree of neuronal impairment in brain cortex as claimed in any of claims 8 to 12, wherein the sensors (2) comprise EEG sensors or MEG sensors.

**14.** The apparatus for measuring the degree of neuronal impairment in brain cortex as claimed in any one of claims 8 to 13, wherein the sensors (2) and the output unit (31, 32) are provided in a terminal equipment, the computing unit is provided in an operation center (42), and the terminal equipment and the operation center are connected through a communication line (41).

**15.** A computer program for measuring the degree of neuronal impairment in brain cortex, and making a computer execute the steps of:

obtaining a dipolarity at each sampling based on numerical data of scalp potentials of a subject measured by mounting a plurality of sensors on the head of the subject;
obtaining mean values of squared errors, within a fixed time interval, between a scalp potential by an equivalent dipole at a dipolarity peak emergence time and the measured scalp potentials, or variances of the squared errors from the mean values for the sensors; and
mapping a contour concerning a distribution of the mean values or the variances on a scalp or a brain surface corresponding thereto, as an output.

**16.** The computer program as claimed in claim 15 wherein the mean values or the variances within the fixed time interval are obtained for an overall time interval, and a contour for the mean values or the variances is mapped as an output.

**17.** The computer program as claimed in claim 15 wherein the dipolarity indicates an approximate degree, after a predetermined frequency component within the data is extracted, and based on the predetermined frequency component, at a time when one or more equivalent dipoles are determined in which a mean value of a squared error between a potential distribution which one or more current dipoles, supposed in the head, form at positions of the sensors and a measured potential of the sensors indicated by the data becomes least.

**18.** The computer program as claimed in claim 15 wherein the head comprises a spherical model.

**19.** A computer program for measuring the degree of neuronal impairment in brain cortex, and making a computer execute the steps of:

measuring scalp potentials by a plurality of sensors mounted on the heads of reference persons to be converted into numerical data;
obtaining squared mean values from the numerical data at the sensors for every short time interval within a designated measurement time;
obtaining a normalized standard deviation of the squared mean values within the designated measurement time;
obtaining the normalized standard deviation for a plurality of reference persons;
obtaining a mean value of the normalized standard deviations and an inter-reference person standard deviation for the mean value to be stored; and
determining a distance level of a normalized standard deviation similarly obtained for a subject from the normalized standard deviation mean value of the reference persons based on the inter-reference person standard deviation, made different between a positive side and a negative side of the normalized standard deviation mean value of the reference persons.

**20.** The computer program as claimed in claim 19 wherein levels are projected to positions, on a brain surface, preliminarily obtained corresponding to sensor positions on a scalp, and by interpolating the levels, a contour concerning a distribution on the brain surface is mapped and colored.

**21.** A computer readable recording medium for recording the program as claimed in any one of claims 15 to 20.

# FIG.1

CALCULATE MEAN VALUE OF SQUARED ERRORS AT RESPECTIVE PEAKS
(PER EACH SENSOR)

PEAK P1  P2  P10 ······ P20 ······ P30 ··· P40 ······ P50 ······ P60 ··· P69 P70

DIPOLARITY

TIME (SEC)

SINGLE TIME INTERVAL (5 SECONDS = 512 SAMPLES)

FIG.2

## FIG.3A

### NORMAL SUBJECT

## FIG.3B

### ABNORMAL SUBJECT

## FIG.3C

### SPECT IMAGE

## FIG.4

# FIG.5

EP 1 447 045 A1

# FIG.6

ARRANGE SENSORS AT DESIGNATED POSITIONS OF HEAD REGARDED AS SPHERICAL CONDUCTOR — S1

INITIALIZE — S2

READ PROGRAM FROM EXTERNAL STORAGE TO BE STORED IN MEMORY — S3

MEASURE POTENTIAL BY SENSOR — S4

SEPARATE BRAIN WAVE COMPONENT HAVING PEAK IN SPECIFIED FREQUENCY BANDWIDTH SUCH AS α WAVE BY DIGITAL FILTERING — S5

DESIGNATE OVERALL SAMPLING TIME INTERVAL FOR MAPPING — S6

S13
OUTPUT CONTOUR MAP FOR EVERY 5 SECONDS

S7
IS OVERALL SAMPLING TIME INTERVAL (2 MINUTES) EXPIRED?

YES

NO

S12
CALCULATE MEAN VALUES FOR SENSORS IN OVERALL TIME INTERVAL & VARIANCE CONCERNING FLUCTUATION, AND PREPARE & OUTPUT CONTOUR MAP CONCERNING DISTRIBUTION ON SCALP OR BRAIN SURFACE CORRESPONDING THERETO

CALCULATE SINGLE EQUIVALENT DIPOLE FROM MEASURED SCALP POTENTIAL AT EACH SAMPLING, & RECORD SQUARED ERRORS BETWEEN SCALP POTENTIAL BY EQUIVALENT DIPOLE OBTAINED THEN & MEASURED POTENTIAL FOR EACH SENSOR — S8

CALCULATE DIPOLARITY OF SINGLE EQUIVALENT DIPOLE, & DETECT PEAK EMERGENCE TIME THEREIN — S9

CALCULATE MEAN VALUE OF SAID SQUARED ERRORS FOR EACH SENSOR AT DIPOLARITY PEAK EMERGENCE TIME WITHIN SINGLE SAMPLING TIME INTERVAL (E.G. 5 SECONDS) & VARIANCE CONCERNING FLUCTUATION OF SQUARED ERRORS — S10

INTERPOLATE CONCERNING MEAN VALUES & VARIANCES AT SENSOR VALUES BY E.G. SPLINE CURVE, AND PREPARE & STORE CONTOUR MAP CONCERNING DISTRIBUTION ON SCALP OR BRAIN SURFACE CORRESPONDING THERETO — S11

## FIG.7

RECORD POTENTIAL $u_j(t)$ OF j SENSOR OF REFERENCE PERSON — S21

EXTRACT 5-15 Hz COMPONENT — S22

S23

CALCULATE SQUARED MEAN VALUE FOR EVERY $\tau$ (2) SECONDS

$< u_j(0)^2 >, < u_j(\tau)^2 >, < u_j(2\tau)^2 >, \cdots < u_j(60\tau)^2 >,$

WHERE $<u_j(\tau)^2> = \dfrac{1}{\tau}\int_{\tau}^{2\tau} u_j(t)^2 dt, \cdots$ Eq (1)

MEAN VALUE $m_j$

TIME

$0 \quad \tau \quad 2\tau \quad 3\tau$

S24

OBTAIN STANDARD DEVIATION $\sigma_{js}$ AROUND MEAN VALUE $m_j$ OF $< u_j(0)^2 >, < u_j(\tau)^2 >, \cdots, \&$ DIVIDE IT BY $m_j$, THEREBY CALCULATING NORMALIZED STANDARD DEVIATION $\sigma_j$

S25

CALCULATE MEAN VALUE $S_j$ OF $\sigma_j$ & STANDARD DEVIATION $s_j$ AROUND $S_j$ FOR NUMEROUS REFERENCE PERSONS

DISTRIBUTION OF $\sigma_j$ CONCERNING REFERENCE PERSON

$s_j$

$0 \qquad S_j \qquad \sigma_j$

S26

OBTAIN $(S_1, s_1), (S_2, s_2), \cdots, (S_{21}, s_{21})$ FOR ALL 21 SENSORS TO BE STORED AS DATABASE

S27

CALCULATE NORMALIZED STANDARD DEVIATION $\Sigma_j$ FOR SUBJECT LIKE ABOVE

S28

DETERMINE DISTANCE LEVEL OF NORMALIZED STANDARD DEVIATION $\Sigma_j$ OF SUBJECT FROM NORMALIZED STANDARD DEVIATION MEAN VALUE $S_j$ OF REFERENCE PERSON BY STANDARD DEVIATION $s_j$ (DIFFERENTIATE LEVELS DEPENDING ON LEFT OR RIGHT OF $S_j$)

STANDARD DEVIATION OF REFERENCE PERSON

$s_j$

| -2 (6) | -1 (5) | 0 (4) | 0 (1) | +1 (2) | +2 (3) |

$0 \qquad S_j \quad \Sigma_j \qquad \sigma_j$

S29

INTERPOLATE VALUES OF NORMALIZED STANDARD DEVIATION $\Sigma_j$ AT EACH LEVEL, AND PREPARE & COLOR CONTOUR MAP CONCERNING DISTRIBUTION ON BRAIN SURFACE CORRESPONDING TO SENSOR POSITION ON SCALP PRELIMINARILY OBTAINED

# FIG.8

2 SENSOR

8 PROJECT POINT OF SENSOR

6 BRAIN

7 CENTER OF HEAD

1 HEAD

## FIG.9A

LEFT SIDE VIEW OF BRAIN
(LEFT SIDE IS FRONT)

## FIG.9B

RIGHT SIDE VIEW OF BRAIN
(RIGHT SIDE IS FRONT)

## FIG.9C

TOP VIEW OF BRAIN
(UPPER SIDE IS FRONT)

**European Patent Office**

## PARTIAL EUROPEAN SEARCH REPORT

**Application Number**

which under Rule 45 of the European Patent Convention
shall be considered, for the purposes of subsequent
proceedings, as the European search report

EP 03 02 8545

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br>Y<br>A | EP 1 216 656 A (BRAIN FUNCTIONS LAB INC) 26 June 2002 (2002-06-26)<br><br>* the whole document *<br>--- | 11,13, 14,19,21<br>12,20<br>1-3, 6-10, 15-18 | A61B5/0476<br>A61B5/04 |
| Y<br><br>A | US 4 949 725 A (RAVIV GIL ET AL) 21 August 1990 (1990-08-21)<br>* abstract *<br>--- | 12,20<br><br>1,2,8,9 | |
| A | MUSHA T ET AL: "A new EEG method for estimating cortical neuronal impairment that is sensitive to early stage Alzheimer's disease"<br>CLINICAL NEUROPHYSIOLOGY, JULY 2002, ELSEVIER, IRELAND,<br>vol. 113, no. 7, pages 1052-1058,<br>XP002278524<br>ISSN: 1388-2457<br>* 2. Methods p. 1053-1055 *<br>--- | 1,8,11, 15,19 | |
| | -/-- | | TECHNICAL FIELDS SEARCHED (Int.Cl.7)<br>A61B |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

1-3,8-21

Claims searched incompletely :

6,7 (when depending on 4)

Claims not searched :

4,5

Reason for the limitation of the search:

Article 52 (4) EPC - Diagnostic method practised on the human or animal body

| Place of search | Date of completion of the search | Examiner |
|---|---|---|
| BERLIN | 5 May 2004 | Trachterna, M |

EPO FORM 1503 03.82 (P04C07)

**European Patent Office**

**PARTIAL EUROPEAN SEARCH REPORT**

Application Number

EP 03 02 8545

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | TAKE N ET AL: "Three-dimensional display for multi-sourced activities and their relations in the human brain by information flow between estimated dipoles" MEDICAL IMAGE COMPUTING AND COMPUTER-ASSISTED INTERVENTION - MICCAI 2002. 5TH INTERNATIONAL CONFERENCE. PROCEEDINGS, PART II (LECTURE NOTES IN COMPUTER SCIENCE VOL.2489), pages 93-100, XP002278525 2002, Berlin, Germany, Springer-Verlag, Germany ISBN: 3-540-44225-1 * abstract * * 2. Three Dimensional Display Tool p. 94* ----- | 1-3, 8-12,15, 16,18-20 | |
| | | | **TECHNICAL FIELDS SEARCHED** (Int.Cl.7) |

EPO FORM 1503 03.82 (P04C10)

## ANNEX TO THE EUROPEAN SEARCH REPORT
## ON EUROPEAN PATENT APPLICATION NO.

EP 03 02 8545

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2004

| Patent document cited in search report | | Publication date | Patent family member(s) | | Publication date |
|---|---|---|---|---|---|
| EP 1216656 | A | 26-06-2002 | JP | 2002248087 A | 03-09-2002 |
| | | | CN | 1359656 A | 24-07-2002 |
| | | | EP | 1216656 A1 | 26-06-2002 |
| | | | US | 2002107455 A1 | 08-08-2002 |
| US 4949725 | A | 21-08-1990 | NONE | | |

For more details about this annex : see Official Journal of the European Patent Office, No. 12/82